# EUROPEAN PATENT SPECIFICATION

(11) **EP 1 380 264 B1**
(45) Date of publication and mention of the grant of the patent: **01.12.2004**
(21) Application number: 03014568.4
(22) Date of filing: 07.07.2003
(51) Int. Cl.: A61B 17/00, A61B 18/00, G06F 19/00

(54) **Surgery system**
Chirurgisches System
Système chirgurical

(30) Priority: 09.07.2002 JP 2002200258; 10.03.2003 JP 2003063743
(43) Date of publication of application: 14.01.2004
(73) Proprietor: Olympus Corporation, Shibuya-ku, Tokyo (JP)
(72) Inventor: Takahashi, Hiroyuki, Akishima-shi, Tokyo (JP)
(74) Representative: von Hellfeld, Axel, Dr. Dipl.-Phys.,

(56) References cited:
- US-B1- 6 251 113

## Description

### BACKGROUND OF THE INVENTION

### 1. Field of the Invention

The present invention relates to a surgery system comprising an electric scalpel which performs treatment by high-frequency electric current, ultrasonic treatment equipment which performs treatment by ultrasonic, and so forth.

### 2. Description of the Related Art

In recent years, electric scalpel devices which perform treatment using high-frequency electric current, ultrasonic treatment devices which perform treatment using ultrasonic, and so forth, have been developed and implemented as medical devices for treating affected portions of the body.

Various types of treatment equipment are used for the electric scalpel devices and ultrasonic treatment devices, depending on the affected portion or treatment method. Also, water-supply/suction devices and pneumoperitoneum devices and the like are also used in conjunction in the event of treating an affected portion in the body cavity, for cleaning the affected portion and for carrying out pneumoperitoneum.

A system and driving control setting method and the like for using multiple types of treatment equipment such as electric scalpel devices, ultrasonic treatment devices, water-supply/suction devices, pneumoperitoneum devices, and the like, in an efficient manner in accordance with the treatment method, has been proposed in, for example, Japanese Unexamined Patent Application Publication No. 2001-178734, Japanese Unexamined Patent Application Publication No. 9-38098, Japanese Unexamined Patent Application Publication No. 2001-346804, and so forth.

Japanese Unexamined Patent Application Publication No. 2001-178734 proposes a surgery system wherein the types of treatment equipment are distinguished, to automatically set the operating parameters of an ultrasonic coagulation incision device, water-supply/suction device, electric scalpel device, pneumoperitoneum, and so forth, in an appropriate manner, and wherein the system comprises a double foot switch connected to the ultrasonic coagulation incision device and a triple foot switch connected to the water-supply/suction device, so that one of the treatment equipment can be controlled by either of the two foot switches.

Japanese Unexamined Patent Application Publication No. 9-38098 discloses an ultrasonic treatment device comprising ultrasonic driving means for supplying driving signals for driving an ultrasonic transducer, a probe for transmitting the ultrasonic vibrations generated by the ultrasonic driving means to the portion to be treated, electric scalpel signal supplying means for supplying electric scalpel signals to the probe, detecting means for detecting electric scalpel signals supplied from electric scalpel signal supplying means to the probe, and control means for controlling on/off of supply of ultrasonic driving to the probe based on the detection results from the detecting means, wherein ultrasonic treatment and electric scalpel treatment can each be used, with simultaneous use of the ultrasonic and electric scalpel forbidden.

Japanese Unexamined Patent Application Publication No. 2001-346804 proposes a surgery system wherein signal output means which output a signal indicating the state of treatment being carried out by an ultrasonic surgery device are provided in an ultrasonic surgery device, and comprises a pneumoperitoneum device having signal input means for performing ventilation action according to signals indicating the treatment state from the ultrasonic surgery device, wherein the ventilation action of the pneumoperitoneum device is synchronously driven by the treatment signals from the ultrasonic surgery device.

However, there are the following problems with the above-described conventional art.

With the surgery system proposed in Japanese Unexamined Patent Application Publication No. 2001-178734, the two foot switches are connected to different devices, the ultrasonic coagulation incision device and the water-supply/suction device, and the foot switch information is read by control units in the mutually independent ultrasonic coagulation incision device and water-supply/suction device, and then transmitted by mutual communication. Accordingly, in the event that two different foot switches are simultaneously turned on due to the timing of a foot switch turning on being offset, both devices show a status error and driving of the devices is stopped.

With the ultrasonic treatment device proposed in Japanese Unexamined Patent Application Publication No. 9-38098, simultaneous output of the ultrasonic output and electric scalpel output is forbidden, so there is the need to connect the output circuits of each one to another and for each to have a detecting circuit for the output circuit of the other device within its own output circuit, which leads to increased costs. Further, in the event that the other device makes an erroneous output, the output of the device being used is shut down since this arrangement has the device detecting the output of the other device to shut down its own output, which is a troublesome situation.

Also, with the surgery system proposed in Japanese Unexamined Patent Application Publication No. 2001-346804, performing venting operations with the pneumoperitoneum device at the time of inserting the trocar causes slack at the puncturing portion, and puncturing cannot be readily performed. In the event that the pneumoperitoneum device is used while performing ultrasonic suction, the amount of air supplied may become less than the sum of the air vented and the amount suctioned (amount of air supplied < amount of air vented + amount suctioned), which causes the abdominal cavity to slack, causing problems such as adversely affecting the surgical field, and so forth.

US 6,251,113 discloses a surgery system to contol a plurality of medical devices according to the preamble of claim 1.

### SUMMARY OF THE INVENTION

It is an object of the present invention to provide a surgery system wherein, during treatment with certain treatment equipment, other medical devices are synchronously operated, or forbidden to synchronously operate, according to the contents of the treatment, so that multiple types of medical treatment can be realized.

The surgery system according to the present invention comprises: a plurality of types of medical devices to which a plurality of types of treatment equipment are connected, the plurality of types of medical devices generating and outputting treatment equipment driving signals corresponding to the connected treatment equipment; a communicating unit provided between the plurality of types of medical devices, for communicating information one with another; a driving control information transmission unit provided to each of the plurality of types of medical devices, for generating driving control information corresponding to the treatment equipment connected to its own medical device, and transmitting the driving control information to other medical devices via the communication unit; a control information determination reply unit provided to each of the plurality of types of medical devices, for receiving driving control information transmitted from other medical devices and making permission/non-permission determination regarding whether or not operations can be made in synchronization with other medical devices, based on the driving control information, and also making reply of the permission/non-permission determination to the originating medical device via the communication unit; and a driving control decision unit provided to each of the plurality of types of medical devices, for deciding the driving control of the medical device to which it belongs, based on the synchronization driving permission/non-permission determination reply from the control information determination reply unit of another medical device.

In the event of the driving control information transmission unit of the system according to the present invention performing driving control of treatment equipment not necessitating synchronized operations with other medical devices, the driving control information transmission unit may perform inclusion transmission of synchronized operations unnecessary information at another medical device, with the other medical device which has received the synchronized operations unnecessary information performing non-permission settings for synchronized operations.

Also, the driving control information transmission unit of the system according to the present invention may transmit driving control information to other medical devices via the communication unit at constant intervals, and in the event that the driving control information transmitted from the driving control information transmission unit of the originating medical device at constant intervals is not received within a stipulated time period, the control information determination reply unit of the other medical device may stop driving control of its own medical device.

With the surgery system according to the present invention, in the event that one medical device is driven and operated in a system wherein multiple medical devices are connected, the driving operation information thereof is transmitted to the other medical devices, and only a medical device which has been permitted to be driven and operated by the other medical device can be synchronously driven and operated therewith. Or, in the event that another medical device receives information that one medical device is being driven and operated, the other medical device can restrict its own driving operation based on that information, so desired devices can be selected and set from the multiple medical devices, according to the contents or method of treatment.

### BRIEF DESCRIPTION OF THE DRAWINGS

Fig. 1 is a block diagram indicating the configuration of an embodiment of the surgery system relating to the present invention;
Fig. 2 is a block diagram indicating the configuration of an electric scalpel device used for the surgery system;
Fig. 3 is a block diagram indicating the configuration of an ultrasonic output device used for the surgery system;
Fig. 4 is a block diagram indicating the configuration of a water-supply/suction device used for the surgery system;
Fig. 5 is a block diagram indicating the configuration of a pneumoperitoneum device used for the surgery system;
Fig. 6 is a time chart describing the operations of the surgery system;
Fig. 7 is a flowchart describing the operations of the surgery system; and
Fig. 8 is a block diagram illustrating the configuration of another embodiment of the surgery system relating to the present invention.

### DESCRIPTION OF THE PREFERRED EMBODIMENTS

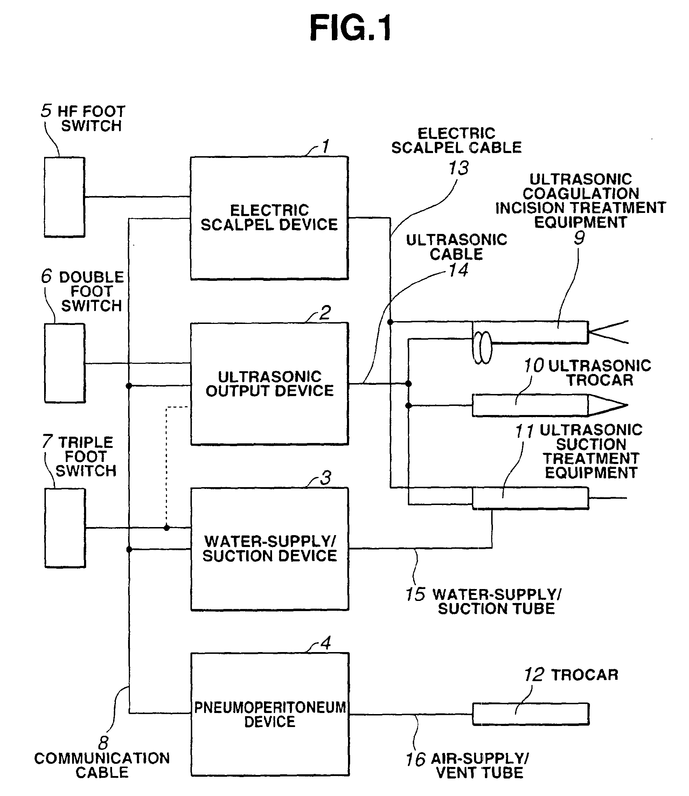

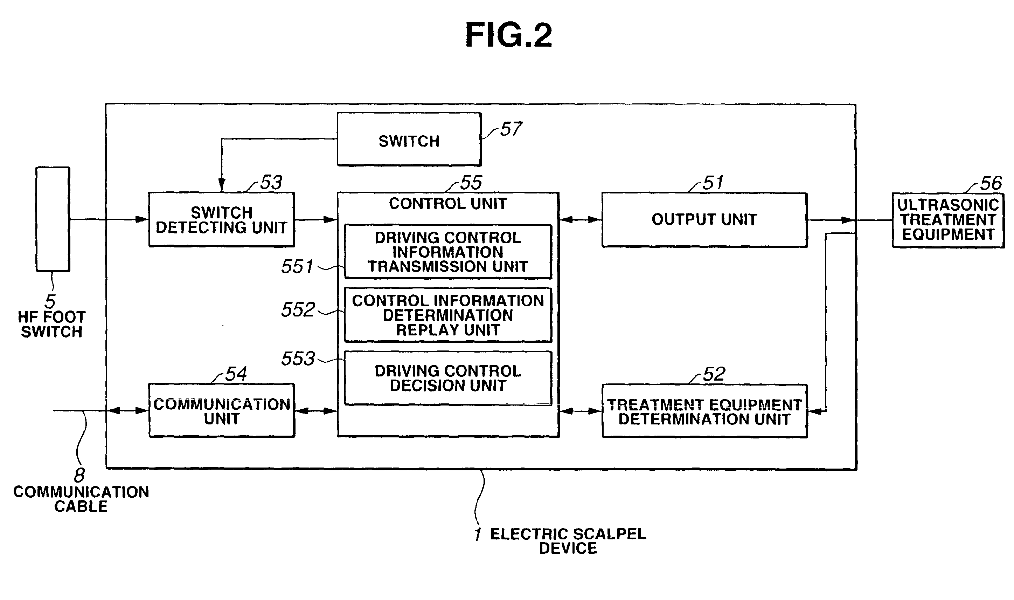

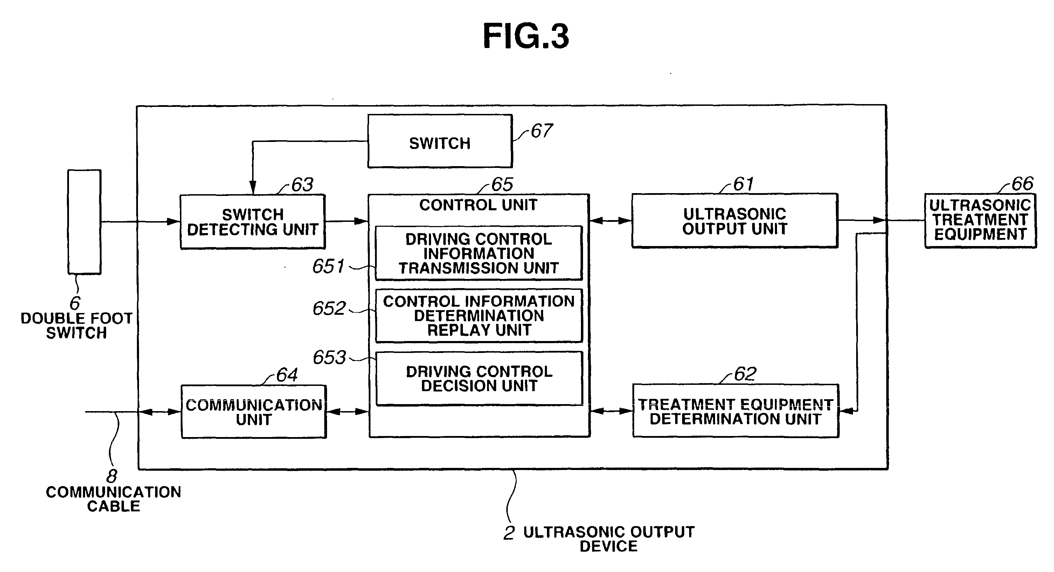

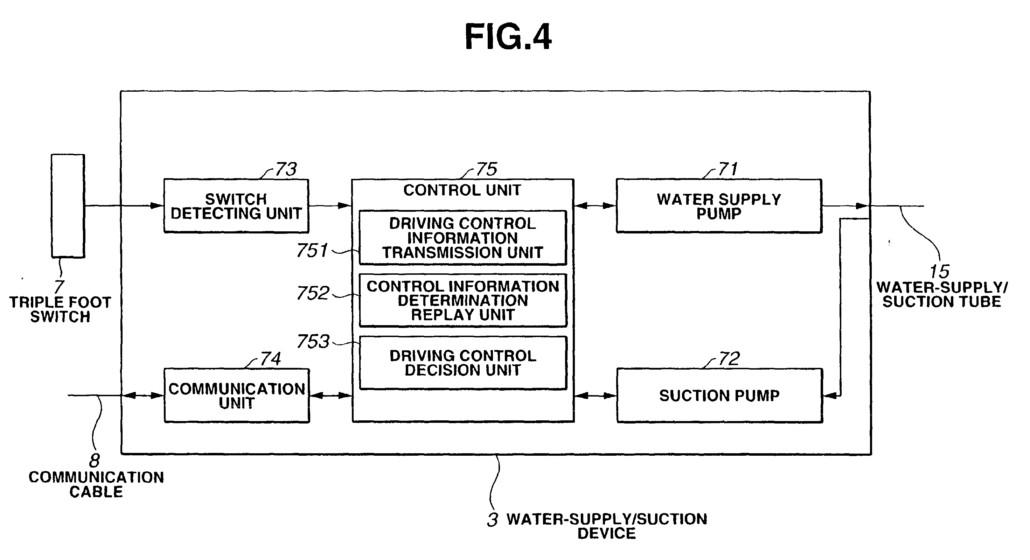

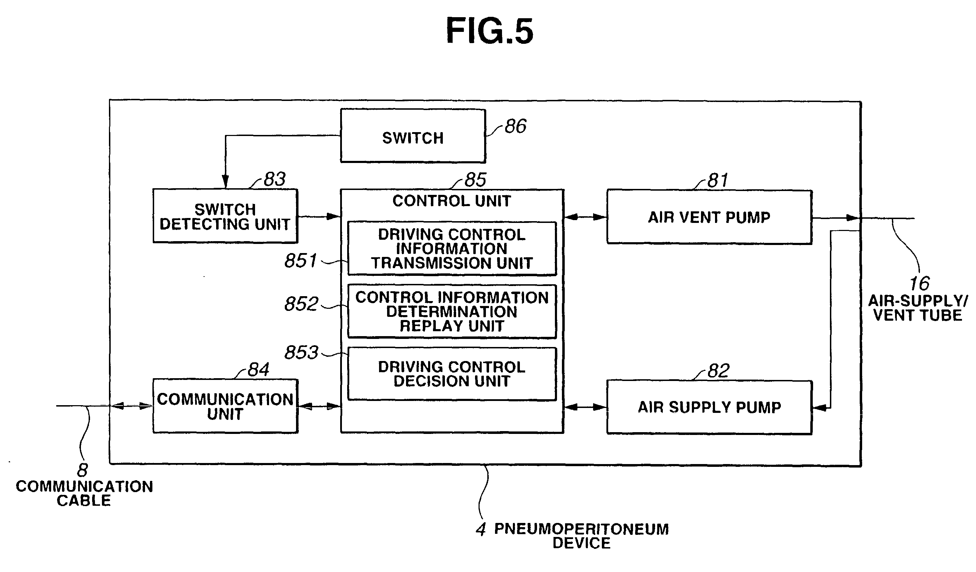

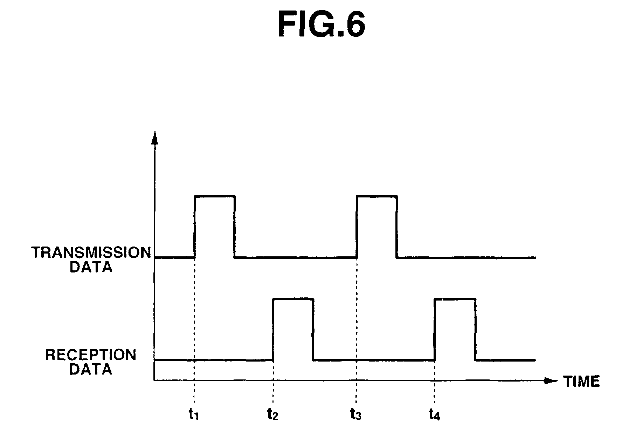

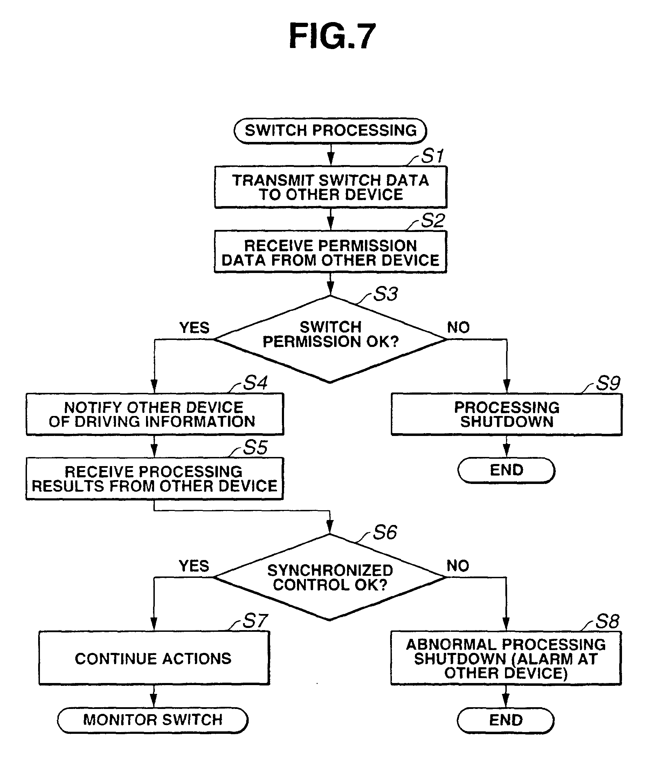

The following is a detailed description of the embodiments of the present invention, with reference to the drawings. A first embodiment of the surgery system according to the present invention will be described with reference to Figs. 1 through 7. Fig. 1 is a block diagram indicating the overall configuration of an embodiment of the surgery system relating to the present invention, Fig. 2 is a block diagram indicating the configuration of an electric scalpel device used for the surgery system, Fig. 3 is a block diagram indicating the configuration of an ultrasonic output device used for the surgery system, Fig. 4 is a block diagram indicating the configuration of a water-supply/suction device used for the surgery system, Fig. 5 is a block diagram indicating the configuration of a pneumoperitoneum device used for the surgery system, Fig. 6 is a time chart describing the operations of the surgery system, and Fig. 7 is a flowchart describing the operations of the surgery system.

As shown in Fig. 1, the surgery system according to the first embodiment of the present invention comprises an electric scalpel device 1 which generates and supplies high-frequency electric current for an electric scalpel to treatment equipment, an ultrasonic output device 2 which generates and supplies ultrasonic vibration driving signals to treatment equipment, a water-supply/suction device 3 which performs water-supply and suction driving of water-supply/suction treatment equipment, and a pneumoperitoneum device 4 which supplies air and vents air to and from the abdominal cavity, with the treatment devices 1 through 4 performing later-described information communication through a communication cable 8.

An HF foot switch 5 for instructing driving of the electric scalpel device 1 is connected to the electric scalpel device 1, and control of the driving output of the electric scalpel device 1 is performed by input of instructions from the HF foot switch 5.

A double foot switch 6 for instructing driving of the ultrasonic output device 2 is connected to the ultrasonic output device 2, and control of the ultrasonic vibration output of the ultrasonic output device 2 is performed by input of instructions from the double foot switch 6.

A triple foot switch 7 for instructing driving of the water-supply/suction device 3 is connected to the water-supply/suction device 3, and driving control of water-supply/suction of the water-supply/suction device 3 is performed by input of instructions from the triple foot switch 7.

Note that input of driving instructions of the ultrasonic output device 2 can be made controlled either from the double foot switch 6 or the triple foot switch 7.

Ultrasonic coagulation incision treatment equipment 9 and ultrasonic suction treatment equipment 11 are connected via an electric scalpel cable 13 on the high-frequency output end of the electric scalpel device 1. The ultrasonic coagulation incision treatment equipment 9, an ultrasonic trocar 10, and the ultrasonic suction treatment equipment 11 are connected to the ultrasonic driving output end of the ultrasonic output device 2 by an ultrasonic cable 14. The ultrasonic suction treatment equipment 11 is connected to the water-supply/suction output end of the water-supply/suction device 3 via a water-supply/suction tube 15. Also, a trocar 12 is connected to the air-supply/vent output end of the pneumoperitoneum device 4 via an air-supply/vent tube 16.

Upon driving instructions being input to the electric scalpel device 1 from the HF foot switch 5, the electric scalpel device 1 supplies high-frequency electric current to the ultrasonic coagulation incision treatment equipment 9 or ultrasonic suction treatment equipment 11 via the electric scalpel cable 13, and treatment such as coagulation or incision of the body tissue is performed by the high-frequency electric current being output from the tips of the ultrasonic coagulation incision treatment equipment 9 or ultrasonic suction treatment equipment 11.

Upon driving instructions being input to the ultrasonic output device 2 from the double foot switch 6, the ultrasonic output device 2 supplies ultrasonic vibration signals to the ultrasonic coagulation incision treatment equipment 9, ultrasonic trocar 10, or ultrasonic suction treatment equipment 11, via the ultrasonic cable 14, whereby ultrasonic coagulation/incision is performed on the body tissue with the ultrasonic coagulation incision treatment equipment 9, the body cavity is bloodlessly punctured from the skin with the ultrasonic trocar 10, and the body tissue is subjected to treatment such as crushing/suction or the like with the ultrasonic suction treatment equipment 11. Note that each of the ultrasonic treatment equipment 9, 10, and 11 have individual ultrasonic transducers, and can generate ultrasonic vibrations upon receiving ultrasonic vibration signals from the ultrasonic output device 2. Or, an arrangement may be made wherein ultrasonic vibrations are output from the ultrasonic output device 2, and supplied to the ultrasonic treatment equipment 9, 10, and 11.

Note that the driving instructions input of the ultrasonic output device 2 may also be carried out from the triple foot switch 7. Accordingly, the two foot switches, i.e., the double foot switch 6 and the triple foot switch 7 contain switches (pedals) capable of at least turning on and off the ultrasonic output.

Upon input of driving instructions from the triple foot switch 7, the water-supply/suction device 3 supplies water and suctions from the tip of the ultrasonic suction treatment equipment 11 via the water-supply/suction tube 15. This water feeding and suctioning action is performed by operating a water feed pedal and suction pedal, not shown in the drawings, which are provided on the triple foot switch 7, thereby supplying cleaning fluid to the body tissue, and recovering and suctioning the cleaning fluid following cleaning the body tissue as well as the crushed body tissue and the like.

In the event of performing treatment by ultrasonic driving of the ultrasonic suction treatment equipment 11 by the ultrasonic output device 2, synchronously driving the water-supply/suction device 3 allows crushing and ultrasonic treatment of the body tissue to be performed while washing the body tissue with the cleaning fluid, and also suctioning the crushed body tissue from the crushing and the ultrasonic treatment to be suctioned along with the cleaning fluid.

The pneumoperitoneum device 4 vents gas within the body cavity from the trocar 12 via the air-supply/vent tube 16. Also, though not shown in the drawings, gas in fed into the body cavity via the air-supply/vent tube 16, thereby performing control to maintain the pneumoperitoneum pressure within the body at a constant level.

Also, as shown in Figs. 2 and 3, the electric scalpel device 1 and the ultrasonic output device 2 are provided with treatment equipment determination units 52 and 62, for determining the treatment equipment connected to the output side. The treatment equipment determination units 52 and 62 identify the treatment equipment connected, such as the ultrasonic coagulation incision treatment equipment 9, ultrasonic trocar 10, and ultrasonic suction treatment equipment 11, and for determining the type of each of the ultrasonic coagulation incision treatment equipment 9, ultrasonic trocar 10, and ultrasonic suction treatment equipment 11. The identification determination of the treatment equipment is performed by providing a resistor with a different value for each type of treatment equipment, or providing a storage medium storing identification information therein, so as to identify the treatment equipment based on the resistance value or the identification information.

That is, as shown in Fig. 2, the electric scalpel device 1 comprises an output unit 51, treatment equipment determination unit 52, switch detecting unit 53, communication unit 54, and control unit 55.

The output unit 51 is for generating and outputting high-frequency electric current for ultrasonic treatment equipment 56 such as the ultrasonic coagulation incision treatment equipment 9 or ultrasonic suction treatment equipment 11 or the like connected to the output unit 51 of the electric scalpel device 1, under driving control from the control unit 55.

The treatment equipment determination unit 52 determines the connection of the ultrasonic treatment equipment 56 such as the ultrasonic coagulation incision treatment equipment 9 or ultrasonic suction treatment equipment 11 connected to the electric scalpel device 1, and the type of the connected ultrasonic treatment equipment 56, so as to output the connection of the ultrasonic treatment equipment 56 and the type information thereof to the control unit 55.

The switch detecting unit 53 detects on/off information of the HF foot switch 5 and various types of operating switches 57 provided to the electric scalpel device 1, so as to output switch information of the HF foot switch 5 and the various types of operating switches being turned on to the control unit 55. Examples of an operating switch 57 include an incision switch and a coagulation switch.

The control unit 55 generates driving control information corresponding to the treatment equipment 56 connected to its own electric scalpel device 1, and also comprises a driving control information transmission unit 551 for transmitting the driving control information to other medical devices via the communication unit 54, a control information determination reply unit 552 for receiving driving control information transmitted from other medical devices and performing permission/non-permission determination regarding synchronized operation with the other medical device based on the driving control information, and returning the permission/non-permission determination to the originating medical device via the communication unit 54, and a driving control decision unit 553 for deciding the driving control of its own electric scalpel device 1 based on the synchronized operation permission/non-permission determination returned from the control information determination reply unit 552 of the other medical device.

The control unit 55 performs driving control of the output unit 51, based on the connection and type information of the ultrasonic treatment equipment 56 provided from the treatment equipment determination unit 52, and the on/off information of the HF foot switch 5 and various types of operating switches 57 from the switch detecting unit 53 and the switch information of a switch being turned on, and generates and outputs high-frequency electric current corresponding to the ultrasonic treatment equipment 56 connected to the output unit 51.

Further, the control unit 55 performs driving control of the communicating unit 54 to generate and transmit via the communication cable 8 the type and driving conditions of the ultrasonic treatment equipment 56 connected to the electric scalpel device 1 and also driving information such as whether synchronized driving with the ultrasonic output device 2, water-supply/suction device 3, and pneumoperitoneum device 4, which are other medical devices, is necessary, and so forth, and also receives driving information from the other medical devices 2 through 4, so as to control the electric scalpel device 1.

Note that the output unit 51 constantly monitors the high-frequency electric current being output to the ultrasonic treatment equipment 56, and in the event of an abnormality, trouble information is sent to the control unit 55, and the supply of the high-frequency electric current is stopped.

As shown in Fig. 3, the ultrasonic output device 2 comprises an ultrasonic output unit 61, treatment equipment determination unit 62, switch detecting unit 63, communication unit 64, and control unit 65.

The ultrasonic output unit 61 is for generating and supplying ultrasonic driving output corresponding to ultrasonic treatment equipment 66 such as the ultrasonic coagulation incision treatment equipment 9, ultrasonic trocar 10, or ultrasonic suction treatment equipment 11 or the like connected to the ultrasonic output unit 61 of the ultrasonic output device 2, under driving control from the control unit 65.

The treatment equipment determination unit 62 determines the connection of the ultrasonic treatment equipment 66 such as the ultrasonic coagulation incision treatment equipment 9, ultrasonic trocar 10, and ultrasonic suction treatment equipment 11 connected to the ultrasonic output unit 61 of the ultrasonic output device 2, and the type of the connected ultrasonic treatment equipment 66, so as to output the connection of the ultrasonic treatment equipment 66 and the type information thereof to the control unit 65.

The switch detecting unit 63 detects on/off information of the double foot switch 6 and various types of operating switches provided to the ultrasonic output device 2, so as to output switch information of the double foot switch 6 and the various types of operating switches being turned on to the control unit 65. Though not shown in the drawings, the switch detecting unit 63 is also capable of detecting on/off of the triple foot switch 7, and information of switches turned on.

The control unit 65 generates driving control information corresponding to the treatment equipment 66 connected to its own ultrasonic output device 2, and also comprises a driving control information transmission unit 651 for transmitting the driving control information to other medical devices via the communication unit 64, a control information determination reply unit 652 for receiving driving control information transmitted from the other medical devices and also performing permission/non-permission determination regarding synchronized operation with the other medical devices based on the driving control information, and returning the permission/non-permission determination to the originating medical device via the communication unit 64, and a driving control decision unit 653 for deciding the driving control of its own ultrasonic output device 2 based on the synchronized operation permission/non-permission determination returned from the control information determination reply unit 652 of the other medical devices.

The control unit 65 performs driving control of the ultrasonic output unit 61, based on the connection and type information of the ultrasonic treatment equipment 66 provided from the treatment equipment determination unit 62, and the on/off information of the double foot switch 6 and various types of operating switches 67 from the switch detecting unit 63 and the switch information of a switch being turned on, and generates and supplies ultrasonic driving output corresponding to the ultrasonic treatment equipment 66 connected to the output unit 61.

Further, the control unit 65 performs driving control of the communicating unit 64 to generate and transmit via the communication cable 8 the type and driving conditions of the ultrasonic treatment equipment 66 connected to the ultrasonic output device 2 and also driving information such as whether synchronized driving with the electric scalpel device 1, water-supply/suction device 3, and pneumoperitoneum device 4, which are other medical devices, is necessary, and so forth, and also receives driving information from the other medical devices 1, 3, and 4, so as to control the driving of the ultrasonic output device 2.

Note that the ultrasonic output unit 61 constantly monitors the ultrasonic driving output being output to the ultrasonic treatment equipment 66, and in the event of an abnormality, trouble information is sent to the control unit 65, and the supply of the ultrasonic driving output is stopped.

As shown in Fig. 4, the water-supply/suction device 3 comprises a water supply pump 71, suction pump 72, switch detecting unit 73, communication unit 74, and control unit 75. The water-supply/suction tube 15 is connected to the water supply pump 71 and suction pump 72.

The switch detecting unit 73 detects the on/off information of the water supply switch and suction switch of the triple foot switch 7, and outputs the water-supply or suction switch information of the switch turned on, to the control unit 75.

The control unit 75 generates driving control information corresponding to the ultrasonic suction treatment equipment 11 connected to its own water-supply/suction device 3, and also comprises a driving control information transmission unit 751 for transmitting the driving control information to the other medical devices via the communication unit 74, a control information determination reply unit 752 for receiving driving control information transmitted from other medical devices and performing permission/non-permission determination regarding synchronized operation with the other medical device based on the driving control information, and returning the permission/non-permission determination to the originating medical device via the communication unit 74, and a driving control decision unit 753 for deciding the driving control of its own water-supply/suction device 3 based on the synchronized operation permission/non-permission determination returned from the control information determination reply unit 752 of the other medical devices.

The control unit 75 drives the water supply pump 71 and/or suction pump 72 based on the on/off information of the triple foot switch 7 from the switch detecting unit 73 and the switch information of the switch turned on.

Also, the control unit 75 sets the driving conditions for the water supply pump 71 and/or suction pump 72, based on the type of the ultrasonic treatment equipment 66 transmitted from the ultrasonic output device 2 and the synchronized driving information, and upon the ultrasonic output switch of the triple foot switch 7 turning on, detects the on information of the switch at the switch detecting unit 73, and transmits ultrasonic driving information to the communication unit 64 of the ultrasonic output device 2 from the communication unit 74 via the communication cable 8, based on the on information of the switch, so as to effect synchronized driving of the ultrasonic output device 2 and the water-supply/suction device 3.

As shown in Fig. 5, the pneumoperitoneum device 4 comprises an air vent pump 81 and an air supply pump 82 connected to the air-supply/vent tube 16, a switch detecting unit 83, communication unit 84, and control unit 85.

The switch detecting unit 83 detects the on/off information of a button switch 86 such as an air-supply button or vent button or the like provided on the operation panel of the pneumoperitoneum device 4, and the switch information of the button switched on, and outputs to the control unit 85.

Upon the switch detecting unit 83 detecting the air supply switch turning on, and the information thereof being output to the control unit 85, the control unit 85 performs driving control of the air supply pump 82. Upon the switch detecting unit 83 detecting the vent switch turning on, and the information thereof being output to the control unit 85, the control unit 85 performs driving control of the air vent pump 81.

The control unit 85 generates driving control information corresponding to the treatment equipment 12 connected to its own pneumoperitoneum device 4, and also comprises a driving control information transmission unit 851 for transmitting the driving control information to the other medical devices via the communication unit 84, a control information determination reply unit 852 for receiving driving control information transmitted from other medical devices and also performing permission/non-permission determination regarding synchronized operation with the other medical device based on the driving control information, and returning the permission/non-permission determination to the originating medical device via the communication unit 84, and a driving control decision unit 853 for deciding the driving control of its own pneumoperitoneum device 4 based on the synchronized operation permission/non-permission determination returned from the control information determination reply unit 852 of the other medical devices.

Further, upon the communication unit 84 receiving instruction information of air supply and/or venting from the electric scalpel device 1 or ultrasonic output device 2 via the communication cable 8, the control unit 85 performs driving control of the air vent pump 81 and the air supply pump 82 based on the instruction information.

Next, the operations of a surgery system configured thus will be described.

For example, in an arrangement wherein the ultrasonic coagulation incision treatment equipment 9 is connected to the ultrasonic output device 2, in the event that driving instructions for the ultrasonic coagulation incision treatment equipment 9 are input by an operation from the operating switches 67, the connection of the ultrasonic coagulation incision treatment equipment 9 and the type thereof are determined by the treatment equipment determination unit 62 of the ultrasonic output device 2, and switching on of the ultrasonic coagulation incision treatment equipment 9 is detected by the switch detecting unit 63. Based on the determination and detection results from the treatment equipment determination unit 62 and the switch detecting unit 63, the control unit 65 performs driving control of the ultrasonic output unit 61, so as to output optimal ultrasonic driving signals to the ultrasonic coagulation incision treatment equipment 9.

In a case of performing treatment by providing ultrasonic driving output to this ultrasonic coagulation incision treatment equipment 9, water supply and suction by the water-supply/suction device 3 is unnecessary, but there is need for ventilation by the pneumoperitoneum device 4 in order to vent the smoke and mist generated by the treatment using the ultrasonic coagulation incision treatment equipment 9 from the body cavity, to keep a clear field of view within the body cavity.

Also, in the case of performing ultrasonic vibration treatment by providing driving control to this ultrasonic coagulation incision treatment equipment 9 from the ultrasonic output device 2, there is the need for supply of the electric scalpel high-frequency output from the electric scalpel device 1 to the ultrasonic coagulation incision treatment equipment 9 to be forbidden. This is to avoid the danger of excessive temperature rise due to applying both the ultrasonic vibration output and electric scalpel high-frequency output to the body tissue from the ultrasonic coagulation incision treatment equipment 9.

To this end, in a case wherein the ultrasonic coagulation incision treatment equipment 9 is connected and driven, the control unit 65 of the ultrasonic output device 2 generates driving information regarding forbidding of synchronized driving of the ultrasonic output device 2 and electric scalpel device 1 or water-supply/suction device 3, and synchronized driving of the ultrasonic output device 2 and the pneumoperitoneum device 4, and so forth, which is transmitted from the communication unit 64 via the communication cable 8.

Upon receiving the driving information from the ultrasonic output device 2, the control unit 55 of the electric scalpel device 1 sets the driving of the electric scalpel device 1 to a stopped state, and controls such that output of electric scalpel high-frequency output is forbidden even in the event that the HF foot switch 5 is operated and the switch detecting unit 53 detects a switch turning on. Also, the control unit 75 of the water-supply/suction device 3 controls such that water-supply/suction action is forbidden even in the event that a water-supply switch or suction switch of the triple foot switch 7 is operated and the switch detecting unit 73 detects a switch turning on.

On the other hand, with the ultrasonic coagulation incision treatment equipment 9 connected to the ultrasonic output device 2, in the event that the ultrasonic output switch of the double foot switch 6 or triple foot switch 7 is operated, and the switch detecting unit 63 detects the switch turned on, the control unit 65 performs driving control of the ultrasonic output unit 61 so that predetermined ultrasonic driving signals are generated and output to the ultrasonic coagulation incision treatment equipment 9 so as to perform ultrasonic coagulation/incision treatment, and also synchronized driving information for driving the pneumoperitoneum device 4 is created synchronously with this ultrasonic coagulation/incision treatment, and is transmitted from the communication unit 64 to the communication unit 84 of the pneumoperitoneum device 4 via the communication cable 8. The control unit 85 of the pneumoperitoneum device 4 drives the air vent pump 81 and the air supply pump 82 based on the synchronized driving information received by the communication unit 84 of the pneumoperitoneum device 4, and vents the smoke and mist generated by the ultrasonic coagulation/incision treatment out of the body cavity by the trocar 12, connected via the air-supply/vent tube 16.

Thus, the pneumoperitoneum device 4 can be driven synchronously with the treatment using the ultrasonic coagulation incision treatment equipment 9 based on driving control from the ultrasonic output device 2 in a sure manner, and driving of the electric scalpel device 1 and water-supply/suction device 3 synchronous with the ultrasonic output device 2 can be forbidden in a sure manner.

Also, with the ultrasonic coagulation incision treatment equipment 9 and ultrasonic suction treatment equipment 11 connected to the electric scalpel device 1, in the event that electric scalpel treatment is performed with one of the ultrasonic coagulation incision treatment equipment 9 or ultrasonic suction treatment equipment 11 under driving control of the electric scalpel device 1, synchronized driving forbidding information is transmitted from the electric scalpel device 1 to the ultrasonic output device 2 and water-supply/suction device 3, and synchronized driving information is transmitted to the pneumoperitoneum device 4, so that driving of the ultrasonic output device 2 and water-supply/suction device 3 is forbidden even in the event that the double foot switch 6 or triple foot switch 7 is operated, and the electric scalpel device 1 is driven by operation instructions input from the HF foot switch 5 alone, and the pneumoperitoneum device 4 is synchronously driven.

Next, with the ultrasonic trocar 10 connected to the ultrasonic output device 2, in the event that the driving instruction switch of the ultrasonic trocar 10 (one of the various types of operating switches 67) is operated, the ultrasonic output device 2 determines the connection and type of the ultrasonic trocar 10 at the treatment equipment determination unit 62, and the control unit 65 performs driving control of the ultrasonic output unit 61 based on the determination results so that optimal ultrasonic driving signals are generated and output.

In a case of performing treatment by providing ultrasonic driving output to the ultrasonic trocar 10, water supply and suction by the water-supply/suction device 3 is unnecessary, but there is need for the abdominal cavity to be bloated in order to puncture with the ultrasonic trocar 10, so there is the need to stop the air-supply/ventilation driving of the pneumoperitoneum device 4 to maintain the pressure of gas within the body cavity constant.

That is, in the event of driving the ultrasonic trocar 10 with the ultrasonic output device 2, the ultrasonic output device 2 transmits synchronized driving information forbidding the water-supply/suctioning driving of the water-supply/suction device 3 and the air-supply/ventilation driving of the pneumoperitoneum device 4, and upon receiving this, the water-supply/suction device 3 and pneumoperitoneum device 4 are forbidden to perform synchronous driving with the ultrasonic trocar 10 driven and controlled by the ultrasonic output device 2.

Also, while the ultrasonic trocar 10 is being driven and controlled by the ultrasonic output device 2, the ultrasonic trocar 10 is not connected to the electric scalpel device 1, so there is no transmission of the driving information forbidding synchronized driving from the water-supply/suction device 3 to the electric scalpel device 1, and the electric scalpel device 1 execute electric scalpel treatment of the other portions with the ultrasonic coagulation incision treatment equipment 9 or ultrasonic suction treatment equipment 11 connected thereto by operating input by the HF foot switch 5.

That is to say, the control unit 65 of the ultrasonic output device 2 generates driving information forbidding synchronized driving of the water-supply/suction device 3 and pneumoperitoneum device 4 at the time of the ultrasonic trocar 10 being connected and driven, and transmits this via the communication cable 8. Upon receiving the driving information from the ultrasonic output device 2, the control unit 75 of the water-supply/suction device 3 does not drive the water supply pump 71 and suction pump 72 even in the event that turning on of the water supply switch or suction switch of the triple foot switch 7 is detected at the switch detecting unit 73, and the control unit 85 of the pneumoperitoneum device 4 performs control such that the air vent pump 81 and the air supply pump 82 are not driven even in the event that the switch detecting unit 83 detects the driving instruction switch of the pneumoperitoneum device 4 being turned on.

Next, in the event that the ultrasonic suction treatment equipment 11 is connected to the ultrasonic output device 2, the driving instruction switch of the ultrasonic suction treatment equipment 11 (one of the various types of switches 67) is operated, and the switch detecting unit 63 detects the switch turned on, the ultrasonic output device 2 determines the connection and type of the ultrasonic suction treatment equipment 11 at the treatment equipment determination unit 62, and the control unit 65 performs driving control of the ultrasonic output unit 61 based on the determination results and the detection of the switch turned on for the ultrasonic suction treatment equipment 11 by the switch detecting unit 63, so as to output optimal ultrasonic driving signals corresponding to the ultrasonic suction treatment equipment 11. At the time of performing treatment by providing ultrasonic driving output to the ultrasonic suction treatment equipment 11, there is the need for water supply and suction synchronized driving by the water-supply/suction device 3, but synchronized driving of the pneumoperitoneum device 4 is not necessary.

That is to say, forbidding synchronized driving of the pneumoperitoneum device 4 synchronously with the treatment driving of the ultrasonic suction treatment equipment 11 by the ultrasonic output device 2 keeps the suctioning functions of the ultrasonic suction treatment equipment 11 and air-supply/venting functions of the pneumoperitoneum device 4 from being carried out simultaneously. Consequently, the bloating of the abdominal cavity necessary for ultrasonic suction processing can be maintained.

In other words, bloating of the abdominal cavity can be balanced to a predetermined degree between the amount of suction by the ultrasonic suction treatment equipment 11 and the amount of air fed into the abdominal cavity by the pneumoperitoneum device 4. However, in the event that the venting functions of the pneumoperitoneum device 4 are driven and the sum of the air volume displacement of the pneumoperitoneum device 4 and the suction amount of the ultrasonic suction treatment equipment 11 exceeds the amount of air supplied by the pneumoperitoneum device 4, the abdominal cavity shrinks, and treatment with the ultrasonic suction treatment equipment 11 becomes difficult.

Now, driving control of the ultrasonic coagulation incision treatment equipment 9 or ultrasonic suction treatment equipment 11 with the electric scalpel device 1 while driving control of the ultrasonic suction treatment equipment 11 with the ultrasonic output device 2 will obstruct treatment due to increased heat and so forth, so driving information forbidding driving is transmitted from the ultrasonic output device 2 to the electric scalpel device 1, and the electric scalpel device 1 stops the electric scalpel output even in the event of detecting operating input by the HF foot switch 5, based on the driving information forbidding driving.

Next, the communication operations of the driving information carried out over the communication cable 8 between the medical devices of the surgery system according to the present invention will be described with reference to the time chart shown in Fig. 6. Fig. 6 illustrates a transmission data and reception data time chart for a certain originating medical device.

At time t1, in the event that there is operational input from the double foot switch 6 or triple foot switch 7, to the ultrasonic output device 2 which is the originating medical device, for driving the ultrasonic coagulation incision treatment equipment 9, the ultrasonic output device 2 generates driving instruction information for the ultrasonic coagulation incision treatment equipment 9, driving information for the pneumoperitoneum device 4 to perform venting driving synchronously with the ultrasonic coagulation incision treatment equipment 9, and driving information forbidding driving of the electric scalpel device 1 and the water-supply/suction device 3, and transmits this as transmission data to the electric scalpel device 1, water-supply/suction device 3, and pneumoperitoneum device 4, which are receiving medical devices, via the communication cable 8.

The electric scalpel device 1, water-supply/suction device 3, and pneumoperitoneum device 4, which are receiving devices, which have received the driving information transmitted from the ultrasonic output device 2, return information with regard to whether an operating switch has been turned on and the device is operated, or an operating switch has not been turned on and the device is not being operated, prior to receiving the driving information from the ultrasonic output device 2 which is the originating device.

That is to say, for example, a medical device regarding which an operating switch is turned on and is driven and controlled (hereafter referred to as "originating medical device") generates and transmits driving information such as the identification of the medical device switched on, the type of the switch operated, the identification of the treatment equipment to be driven, the identification of the medical devices driven synchronously, and identification of medical devices regarding which synchronized driving is forbidden (hereafter referred to as "driving information transmission from originating medical device"). A medical device which has received the driving information transmission from the originating medical device (hereafter referred to as "receiving medical device") returns information to the originating medical device regarding whether or not the device had been driven by an operating switch before receiving the driving information (hereafter referred to as "driving information reply from receiving medical device").

Next, at time t2, the originating medical device receives a message regarding whether or not a switch of the receiving medical device has been operated, as reception data.

That is to say, in the event that the driving information reply from the receiving medical device shows that the receiving medical device has been switched on and is being driven and controlled before transmission of the driving information from the originating medical device, the receiving medical device performs processing to invalidate the driving information transmitted from the originating medical device and returns a message to that effect to the originating medical device. On the other hand, in the event that the receiving medical device has not been switched on and is not being driven and controlled at any way at the point of transmission of the driving information from the originating medical device, the receiving medical device performs processing to validate the driving information transmitted from the originating medical device, and returns a message to that effect to the originating medical device.

Next, at time t3, the valid/invalid results of the driving information of the originating medical device from the receiving medical device received at time t2, is transmitted to other receiving medical device connected thereto as transmission data. That is, whether the switch of the originator has been validated or invalidated, is transmitted. At the medical device receiving the message, control settings such as synchronized driving or forbidding thereof of the receiving medical device, and input restriction (forbidding) of operating switches, is performed based on the message.

Further, at time t4, the originating medical device receives driving information such as the synchronized driving or forbidding thereof, and input restriction (forbidding) of operating switches set to the other receiving medical devices, as reception data of the receiving medical device, and finalizes the control settings.

Accordingly, in the event operating input for driving the ultrasonic coagulation incision treatment equipment 9 is made from the foot switches 6 or 7 to the ultrasonic output device 2, for example, selecting of other medical devices to be driven synchronously with the driving of the ultrasonic output device 2 or regarding which synchronized driving is to be forbidden, and setting of driving conditions, can be performed unless the electric scalpel device 1, water-supply/suction device 3, and pneumoperitoneum device 4, which are other medical devices, are already being operated and driven.

The actions of the surgery system will be described in detail with reference to the flowchart shown in Fig. 7. In step S1, the medical device regarding which input instructing operations has been made by an operating switch transmits the driving information of the medical device to be switched on, to other medical devices. That is, driving information of the originating medical device is transmitted from the originating medical device to the receiving medical device.

Upon receiving in step S1 the driving information of the medical device switched on, which is the originating medical device, in step S2, the other medical device which is the receiving medical device generates permission data regarding whether or not driving control has been made by another input switch before receiving that driving information, i.e., regarding whether or not driving information from the originating medical device can be accepted, which is returned to the originating medical device.

In step S3, the originating medical device determines whether driving information from the originating medical device has been received at the receiving medical device, based on the permission data from the receiving medical device in step S2.

In the event that the determination in step S3 shows that receiving of driving information of the originating medical device is permitted by the receiving medical device, step S4 and succeeding steps are executed. On the other hand, in the event that the receiving medical device is determined to be already driven under input instructions of an operating switch, so that driving under the driving information from the originating medical device is not permitted, the driving of the originating medical device is stopped in step S9.

In the event that acceptance of the driving information of the originating medical device is permitted at the receiving medical device in step S3, in step S4, the originating medical device transmits driving information for synchronized driving and forbidding synchronized driving, to the receiving medical device. In step S5, the receiving medical device receives the driving information for synchronized driving and forbidding synchronized driving transmitted from the originating medical device, and returns signals indicating the reception to the originating medical device.

Next, in step S6, the originating medical device determines whether or not the signals received from the receiving medical device in step S5 are signals indicating synchronized driving information, and in the event that determination is made that the synchronized driving information has been received, in step S7 the originating medical device performs continued operation of the receiving medical device synchronously with the driving of the originating medical device. Turning on and off of switches is monitor while the operations are being performed in step S7, the turning on and off of the switches is consecutively read, and whether or not to stop the operations with the switch is distinguished.

Also, in the event that determination is made in step S6 by the originating medical device that the signals indicate that the receiving medical device has not received the synchronized driving information, abnormal stopping processing of the originating medical device and the receiving medical device is performed in step S8, since trouble is assumed to have occurred at the receiving medical device. At this time, the receiving medical device rejecting synchronized driving can be identified by making an abnormal display on the receiving medical device which has not received the synchronized driving information.

As described above, with the surgery system according to the present invention, multiple types of treatment equipment can be connected to multiple types of medical devices, wherein treatment equipment operated according to the content of the treatment are driven with priority, and settings for synchronized driving or forbidding synchronized driving with the other medical devices and treatment equipment are made, so a surgery system which is easy to operate, readily expandable, and capable of handling multiple types of treatment equipment, can be provided.

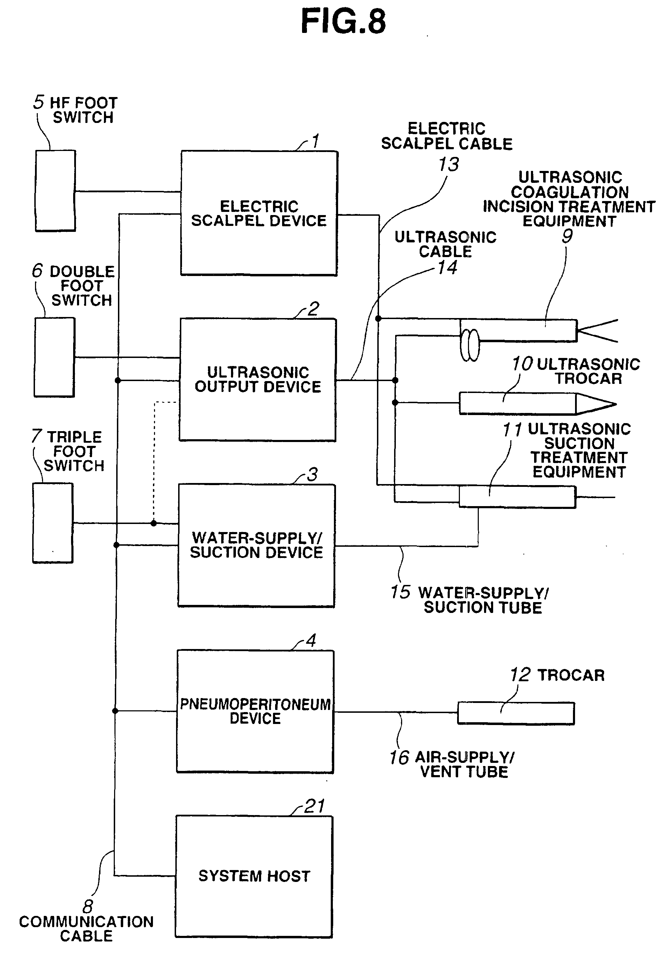

Next, the surgery system according to another embodiment of the present invention will be described with reference to Fig. 8. Note that the portions which are the same as those shown in Fig. 1 are denoted with the same reference numerals, and detailed description thereof will be omitted.

The surgery system shown in Fig. 8 comprises a system host 21 in the surgery system described with reference to Fig. 1 for performing driving control of the medical devices such as the electric scalpel device 1, ultrasonic output device 2, water-supply/suction device 3, and pneumoperitoneum device 4, via the communication cable 8.

The system host 21 comprises a microprocessor for performing driving control of the multiple medical devices 1 through 4, having various control sequences, and a touch panel which is a setting screen for inputting and setting various types of driving control instructions and driving control conditions and so forth as to this microprocessor, and also displaying the driving control instructions and driving control conditions.

That is, settings such as selection of which of the ultrasonic coagulation incision treatment equipment 9 and the ultrasonic suction treatment equipment 11 is to supply electric scalpel output to, setting of the electric scalpel output intensity, and so forth, are made to the electric scalpel device 1 from the touch panel. Selection of which of the ultrasonic coagulation incision treatment equipment 9, ultrasonic trocar 10, and the ultrasonic suction treatment equipment 11 the ultrasonic output device 2 is to supply ultrasonic driving signals to, settings of the amount of ultrasonic vibrations of the ultrasonic treatment equipment 9 through 11, and so forth, are made to the ultrasonic output device 2. Settings regarding water-supply/suction operations and the amount of water-supply/suction are made to the water-supply/suction device 3. Settings regarding the air supply and venting, the amount of air supply and venting, and turning the air supply and venting on and off, is made to the pneumoperitoneum device 4.

Based on the conditions set and input from the touch panel, the microprocessor performs the desired treatment by performing driving control such as synchronously driving the treatment equipment 9 through 12 via the various types of medical devices 1 through 4, or forbidding synchronized driving, following a predetermined control sequence.

Thus, driving conditions, such as the driving conditions of the various types of medical devices 1 through 4 and synchronized driving or forbidding synchronized driving, are set, from the system host 21 via the communication cable 8, and the desired treatment equipment 9 through 12 is driven by operating the foot switches 5 through 7 under the set driving conditions, thereby enabling medical treatment.

In the above-described surgery system according to the present invention, with regard to the driving information exchanged via the communication cable 8 between the various types of medical devices 1 through 4 shown in Fig. 1, or the exchange of driving conditions information via the communication cable 8 between the various types of medical devices 1 through 4 and the system host 21 shown in Fig. 8, electric scalpel output or ultrasonic vibration signals must be continuously output while the affected portion is being treated using the ultrasonic coagulation incision treatment equipment 9 for example, under the control of the electric scalpel device 1 or the ultrasonic output device 2, i.e., while the foot switch 5 or 6 is on, and also, the water supply, suction, air supply, and venting must each be continuously driven while the water supply switch or suction switch of the water-supply/suction device 3, or the air supply switch or venting switch of the pneumoperitoneum device 4 are on.

Accordingly, reliability of information communication must be ensured between the medical devices 1 through 4 and between the medical devices 1 through 4 and the system host 21 over the communication cable 8.

To this end, the originator of driving information of the various types of medical devices 1 through 4, or the system host 21, transmit driving information at predetermined time intervals, e.g., once every second. On the other hand, the receiver of the of driving information of the various types of medical devices 1 through 4 maintain driving under the driving information being transmitted at the constant intervals. In the event that the driving information being transmitted at the constant time intervals cannot be received at one of the receiving medical devices 1 through 4 for a predetermined amount of time, an assumption is made that some sort of trouble has occurred at one of the medical devices 1 through 4 serving as the driving information originator or the system host 21, and the driving operations are stopped.

Thus, the reliability between the medical devices, treatment equipment, and system host can be insured for mutual information communication, and in the event that trouble occurs, the operations of the various types of medical devices can be speedily stopped, so as to quickly switch to a different surgery system.

The surgery system of the present invention is advantageous in that multiple medical devices and multiple pieces of treatment equipment connected to the medical devices can be used, the medical devices and treatment equipment optimal for the contents of treatment and treatment method can be selected, and the identification of the medical devices to be synchronously driven according to the selected medical devices and treatment equipment and the medical devices to forbid synchronized driving can be automatically set, thereby improving the efficiency of surgery.

Having described the preferred embodiments of the invention referring to the accompanying drawings, it should be understood that the present invention is not limited to those precise embodiments and various changes and modification thereof could be made by one skilled in the art without departing from the scope of the invention as defined in the appended claims.

## Claims

1. A surgery system comprising:
a plurality of types of medical devices (1, 2, 3, 4) to which a plurality of types of treatment equipment (9, 10, 11) are connected, the plurality of types of medical devices generating and outputting treatment equipment driving signals corresponding to the connected treatment equipment; and
communicating means (54, 64, 74, 84) provided between the plurality of types of medical devices (1, 2, 3, 4), for communicating information one with another, the surgery system comprising:
driving control information transmission means provided to each of the plurality of types of medical devices (1, 2, 3, 4), for generating driving control information corresponding to the treatment equipment connected to its own medical device, and also transmitting the driving control information to other medical devices via the communication means; **characterised in that** the surgery system further comprises:
control information determination reply means provided to each of the plurality of types of medical devices (1, 2, 3, 4), for receiving driving control information transmitted from other medical devices and also making permission/non-permission determination regarding whether or not operations can be made in synchronization with other medical devices, based on the driving control information, and also making reply of the permission/non-permission determination to the originating medical device via the communication means; and
driving control decision means provided to each of the plurality of types of medical devices (1, 2, 3, 4), for deciding the driving control of the medical device to which it belongs, based on the synchronization driving permission/non-permission determination reply from the control information determination reply means of another medical device.

2. A surgery system according to Claim 1, wherein in the event of the driving control information transmission means performing driving control of treatment equipment not necessitating synchronized operations with other medical devices, the driving control information transmission means performs inclusion transmission of synchronized operations unnecessary information at another medical device, and wherein the other medical device which has received the synchronized operations unnecessary information performs non-permission settings for synchronized operations.

3. A surgery system according to Claim 1, wherein the driving control information transmission means transmits driving control information to other medical devices via the communication means at constant intervals, and in the event that the driving control information transmitted from the driving control information transmission means of the originating medical device at constant intervals is not received within a stipulated time period, the control information determination reply means of the other medical device stops driving control of its own medical device.

4. A surgery system according to Claim 3, wherein the driving control information is switch data indicating that an operating switch connected to the originating medical device has been switched on or off.

5. A surgery system according to Claim 1, wherein the plurality of types of medical devices (1, 2, 3, 4) include an electric scalpel device (1) for supplying high-frequency electric current to treatment equipment, an ultrasonic output device (2) for supplying ultrasonic driving signals to the treatment equipment, a water-supply/suction device (3) for supplying cleaning water and the like to the treatment equipment and suctioning water therefrom, and a pneumoperitoneum device (4) for supplying air to the treatment equipment and venting air therefrom.

6. A surgery system according to Claim 5, the electric scalpel device (1) comprising:
output means (51) for generating and outputting high-frequency electric current corresponding to the treatment equipment connected thereto;
treatment equipment determining means (52) for determining the connection and type of treatment equipment connected;
switch detection means (53) for detecting an operating switch for the connected treatment equipment being switched on;
control means (55) for performing driving control of the output means based on
the determination results of the connection and type of the treatment equipment made by the treatment equipment determining means, and
detection of a switch being switched on by the switch detection means; and
communication means (54) for sending driving control information of the electric scalpel device and synchronized driving information with the electric scalpel device to other medical devices, and also receiving driving control information and synchronized driving information from other medical devices, under the control of the control means.

7. A surgery system according to Claim 5, the ultrasonic output device (2) comprising:
output means (61) for outputting ultrasonic driving signals corresponding to the treatment equipment connected thereto;
treatment equipment determining means (62) for determining the connection and type of treatment equipment connected;
switch detection means (63) for detecting an operating switch for the connected treatment equipment being switched on;
control means (65) for performing driving control of the output means based on
the determination results of the connection and type of the treatment equipment made by the treatment equipment determining means, and
detection of a switch being switched on by the switch detection means; and
communication means (64) for sending driving control information of the ultrasonic output device and synchronized driving information with the ultrasonic output device to other medical devices, and also receiving driving control information and synchronized driving information from other medical devices, under the control of the control means.

8. A surgery system according to Claim 5, the water-supply/suction device (3) comprising:
a water supply pump (71) and a suction pump (72);
switch detection means (73) for detecting a switch for instructing driving action of the water supply pump and suction pump being switched on;
control means (75) for performing driving control of the water supply pump and suction pump based on the driving instructions of the water supply pump and suction pump detected by the switch detection means; and
communication means (74) for sending driving control information of the water-supply/suction device to other medical devices, and also receiving driving control information and synchronized driving information from other medical devices, under the control of the control means.

9. A surgery system according to Claim 5, the pneumoperitoneum device (4) comprising:
an air vent pump (81) and an air supply pump (82);
switch detection means (83) for detecting a switch for instructing driving action of the air supply pump and vent pump being switched on;
control means (85) for performing driving control of the air supply pump and vent pump based on the driving instructions of the air supply pump and vent pump detected by the switch detection means; and
communication means (84) for sending driving control information of the pneumoperitoneum device to other medical devices, and also receiving driving control information and synchronized driving information from other medical devices, under the control of the control means.

## Patentansprüche

1. Chirurgisches System aufweisend:
eine Mehrzahl Typen medizinischer Geräte (1, 2, 3, 4), mit denen eine Mehrzahl Behandlungsausrüstungstypen (9, 10, 11) verbunden ist, wobei die Mehrzahl Typen medizinischer Geräte Treibersignale für die Behandlungsausrüstung entsprechend der angeschlossenen Behandlungsausrüstung erzeugt und ausgibt; und
Kommunikationsmittel (54, 64, 74, 84), die zwischen der Mehrzahl medizinischer Geräte (1, 2, 3, 4) zum Austausch von Informationen untereinander vorgesehen sind, wobei das chirurgische System aufweist:
Übertragungsmittel für Treibersteuerinformationen zu jedem Gerät der Mehrzahl Typen medizinischer Geräte (1, 2, 3, 4) zum Erzeugen von Treibersteuerinformationen entsprechend der mit dem eigenen medizinischen Gerät verbundenen Behandlungsausrüstung sowie zum Übertragen der Treibersteuerinformationen über die Kommunikationsmittel an andere medizinische Geräte;
**dadurch gekennzeichnet, dass** das chirurgische System ferner aufweist:
Mittel zur Antwortbestimmung auf die Steuerinformationen, die an jedes Gerät der Mehrzahl Typen medizinischer Geräte (1, 2, 3, 4) geliefert werden, um von anderen medizinischen Geräten übertragene Treibersteuerinformationen zu empfangen und um außerdem eine Bestimmung auf Basis der Treibersteuerinformationen hinsichtlich der Zulassung/Nichtzulassung, ob Operationen synchron mit anderen medizinischen Geräten ausgeführt werden können oder nicht vorzunehmen sind, und um außerdem eine Antwort der Bestimmung hinsichtlich Zulassung/Nichtzulassung über die Kommunikationsmittel an das veranlassende medizinische Geräte zu senden;
Entscheidungsmittel der Treibersteuerung, die für jedes Gerät der Mehrzahl medizinischer Geräte (1, 2, 3, 4) vorgesehen sind, um die Treibersteuerung des zugehörigen medizinischen Geräts auf Basis der Antwortbestimmung auf die Synchronisierung der Zulassung/Nichtzulassung von den Mitteln zur Antwortbestimmung auf die Steuerinformationen zu entscheiden.

2. Chirurgisches System nach Anspruch 1, bei dem in dem Fall, in dem die Übertragungsmittel für Treibersteuerinformationen eine Treibersteuerung von Behandlungsausrüstung vornimmt, die keine synchronisierten Operationen mit anderen medizinischen Geräten erfordert, die Übertragungsmittel für Treibersteuerinformationen eine bedingte Übertragung synchronisierter Operationen mit unnötigen Informationen an ein anderes medizinisches Gerät ausführen, und bei dem das andere medizinische Gerät, das die synchronisierten Operationen mit unnötigen Informationen erhalten hat, Nichtzulassungseinstellungen für synchronisierte Operationen vornimmt.

3. Chirurgisches System nach Anspruch 1, bei dem die Übertragungsmittel für Treibersteuerinformationen in konstanten Intervallen Treibersteuerinformationen über die Übertragungsmittel an andere medizinische Geräte übertragen, und in dem Fall, in dem die von den Übertragungsmitteln für Treibersteuerinformationen in konstanten Intervallen übertragenen Treibersteuerinformationen des ausgebenden medizinischen Geräts nicht innerhalb einer vorgeschriebenen Zeitspanne empfangen werden, die Mittel zur Antwortbestimmung auf die Steuerinformationen des anderen medizinischen Geräts die Treibersteuerung seines eigenen medizinischen Geräts stoppen.

4. Chirurgisches System nach Anspruch 3, bei dem die Treibersteuerinformationen Umschaltdaten sind, die angeben, dass ein mit dem ausgebenden medizinischen Gerät verbundener Betätigungsschalter ein- oder ausgeschaltet worden ist.

5. Chirurgisches System nach Anspruch 1, bei dem die Mehrzahl Typen medizinischer Geräte (1, 2, 3, 4) ein elektrisches Skalpellgerät (1) zur Lieferung eines hochfrequenten elektrischen Stroms an die Behandlungsausrüstung, ein Ultraschall-Ausgabegerät (2) zur Lieferung von Ultraschall-Treibersignalen an die Behandlungsausrüstung, ein Wasserzufuhr-/Absauggerät (3) zur Lieferung von Reinigungswasser und dgl. an die Behandlungsausrüstung und zum Absaugen des Wassers von dieser und ein Pneumoperitoneum-Gerät (4) zur Lieferung von Luft an die Behandlungsausrüstung und zur Entlüftung derselben enthält.

6. Chirurgisches System nach Anspruch 5, bei dem das elektrische Skalpellgerät (1) aufweist:
Ausgabemittel (51) zur Erzeugung und Ausgabe eines hochfrequenten elektrischen Stroms entsprechend der damit verbundenen Behandlungsausrüstung;
Mittel (52) zur Bestimmung der Behandlungsausrüstung, um die Verbindung und den Typ der angeschlossenen Behandlungsausrüstung zu bestimmen;
Schaltdetektormittel (53) zum Detektieren eines eingeschalteten Betätigungsschalters für die angeschlossene Behandlungsausrüstung;
Steuermittel (55) für die Treibersteuerung der Ausgabemittel auf Basis der Bestimmungsergebnisse der Verbindung und des Typs der Behandlungsausrüstung durch die Mittel zur Bestimmung der Behandlungsausrüstung; und
der Detektion eines eingeschalteten Schalters durch die Schaltdetektormittel; und
Kommunikationsmittel (54) zum Senden von Treibersteuerinformationen des elektrischen Skalpellgeräts und synchronisierte Treiberinformationen mit dem elektrischen Skalpellgerät an andere medizinische Geräte sowie zum Empfangen von Treibersteuerinformationen und synchronisierten Treiberinformationen von anderen medizinischen Geräten unter der Steuerung der Steuermittel.

7. Chirurgisches System nach Anspruch 5, bei dem das Ultraschall-Ausgabegerät (2) aufweist:
Ausgabemittel (61) zur Ausgabe von Ultraschall-Treibersignalen entsprechend der damit verbundenen Behandlungsausrüstung;
Mittel (62) zur Bestimmung der Behandlungsausrüstung, um die Verbindung und den Typ der angeschlossenen Behandlungsausrüstung zu bestimmen;
Schaltdetektormittel (63) zum Detektieren eines eingeschalteten Betätigungsschalters für die angeschlossene Behandlungsausrüstung;
Steuermittel (65) für die Treibersteuerung der Ausgabemittel auf Basis der Bestimmungsergebnisse der Verbindung und des Typs der Behandlungsausrüstung durch die Mittel zur Bestimmung der Behandlungsausrüstung; und
der Detektion eines eingeschalteten Schalters durch die Schaltdetektormittel; und
Kommunikationsmittel (64) zum Senden von Treibersteuerinformationen des elektrischen Skalpellgeräts und synchronisierte Treiberinformationen mit dem Ultraschall-Ausgabegerät an andere medizinische Geräte sowie zum Empfangen von Treibersteuerinformationen und synchronisierten Treiberinformationen von anderen medizinischen Geräten unter der Steuerung der Steuermittel.

8. Chirurgisches System nach Anspruch 5, bei dem das Wasserzufuhr-/Absauggerät (3) aufweist:
eine Wasserförderpumpe (71) und eine Absaugpumpe (72);
Schaltdetektormittel (73) zum Detektieren eines eingeschalteten Schalters, um die Antriebsaktion der Wasserförderpumpe und der Absaugpumpe anzuweisen;
Steuermittel (75) für die Treibersteuerung der Wasserförderpumpe und der Absaugpumpe auf Basis der von den Schaltdetektormitteln erkannten Antriebsanweisungen an die Wasserförderpumpe und die Absaugpumpe; und
Kommunikationsmittel (74) zum Senden von Treibersteuerinformationen des Wasserzufuhr-/Absaugeräts an andere medizinische Geräte sowie zum Empfangen von Treibersteuerinformationen und synchronisierten Treiberinformationen von anderen medizinischen Geräten unter der Steuerung der Steuermittel.

9. Chirurgisches System nach Anspruch 5, bei dem das Pneumoperitoneum-Gerät (4) aufweist:
eine Entlüftungspumpe (81) und eine Luftförderpumpe (82);
Schaltdetektormittel (83) zum Detektieren eines eingeschalteten Schalters, um die Antriebsaktion der Luftförderpumpe und der Entlüftungspumpe anzuweisen; Steuermittel (85) für die Treibersteuerung der Luftförderpumpe und der Entlüftungspumpe auf Basis der von den Schaltdetektormitteln erkannten Antriebsanweisungen an die Luftförderpumpe und die Entlüftungspumpe; und
Kommunikationsmittel (84) zum Senden von Treibersteuerinformationen des Pneumoperitoneum-Geräts an andere medizinische Geräte sowie zum Empfangen von Treibersteuerinformationen und synchronisierten Treiberinformationen von anderen medizinischen Geräten unter der Steuerung der Steuermittel.

## Revendications

1. Système chirurgical comprenant :
une pluralité de types d'appareils médicaux (1, 2, 3, 4) auxquels sont connectés une pluralité de types d'équipements de traitement (9, 10, 11), la pluralité de types d'appareils médicaux générant et transmettant en sortie des signaux d'activation d'équipement de traitement correspondant à l'équipement de traitement connecté ; et
des moyens de communication (54, 64, 74, 84) prévus entre la pluralité de types d'appareils médicaux (1, 2, 3, 4) pour l'échange d'informations entre eux, le système chirurgical comprenant :
des moyens de transmission d'informations de commande d'activation prévus sur chacun de la pluralité de types d'appareils médicaux (1, 2, 3, 4) pour générer les informations de commande d'activation correspondant à l'équipement de traitement connecté à leur propre appareil médical, et pour transmettre également les informations de commande d'activation aux autres appareils médicaux par le biais des moyens de communication ;
**caractérisé en ce que** le système chirurgical comprend en outre :
des moyens de réponse de détermination d'informations de commande prévus sur chacun de la pluralité de types d'appareils médicaux (1, 2, 3, 4) pour recevoir les informations de commande d'activation transmises par d'autres appareils médicaux et pour déterminer la permission/non-permission quant à une exécution des opérations en synchronisation avec d'autres appareils médicaux, en se basant sur les informations de commande d'activation, et pour donner également une réponse quant à la détermination de permission/non-permission à l'appareil médical d'origine par le biais des moyens de communication ; et
des moyens de décision de commande d'activation prévus sur chacun de la pluralité de types d'appareils médicaux (1, 2, 3, 4) pour décider l'activation de l'appareil médical auquel ils appartiennent, en se basant sur la réponse de détermination de permission/non-permission d'activation en synchronisation des moyens de réponse de détermination d'informations de commande d'un autre appareil médical.

2. Système chirurgical selon la revendication 1, dans lequel dans le cas où les moyens de transmission d'informations de commande d'activation commandant l'activation de l'équipement de traitement ne nécessitent pas des opérations synchronisées avec d'autres appareils médicaux, les moyens de transmission d'informations de commande d'activation effectuent la transmission pour inclusion des informations inutiles d'opérations synchronisées au niveau d'un autre appareil médical, et dans lequel l'autre appareil médical qui a reçu les informations inutiles d'opérations synchronisées procède aux réglages de non-permission pour les opérations synchronisées.

3. Système chirurgical selon la revendication 1, dans lequel les moyens de transmission d'informations de commande d'activation transmettent les informations de commande d'activation à d'autres appareils médicaux par le biais des moyens de communication à intervalles constants, et dans le cas où les informations de commande d'activation transmises par les moyens de transmission d'informations de commande d'activation de l'appareil médical d'origine à intervalles constants ne sont pas reçues dans une période de temps stipulée, les moyens de réponse de détermination des informations de commande de l'autre appareil médical arrêtent la commande d'activation de leur propre appareil médical.

4. Système chirurgical selon la revendication 3, dans lequel les informations de commande d'activation sont des données de commutation indiquant qu'un interrupteur de fonctionnement connecté à l'appareil médical d'origine a été allumé ou éteint.

5. Système chirurgical selon la revendication 1, dans lequel la pluralité de types d'appareils médicaux (1, 2, 3, 4) comprend un dispositif de scalpel électrique (1) pour fournir un courant électrique haute-fréquence à l'équipement de traitement, un dispositif de production d'ultrasons (2) pour fournir des signaux d'activation ultrasonores à l'équipement de traitement, un dispositif d'alimentation en eau/d'aspiration (3) pour fournir de l'eau de nettoyage et similaire à l'équipement de traitement et pour aspirer de l'eau de celui-ci, et un dispositif de pneumopéritoine (4) pour fournir de l'air à l'équipement de traitement et expulser de l'air de celui-ci.

6. Système chirurgical selon la revendication 5, le dispositif de scalpel électrique (1) comprenant :
des moyens de sortie (51) pour générer et transmettre en sortie un courant électrique haute-fréquence correspondant à l'équipement de traitement connecté à ceux-ci ;
des moyens de détermination de l'équipement de traitement (52) pour déterminer la connexion et le type d'équipement de traitement connecté ;
des moyens de détection de commutation (53) pour détecter qu'un interrupteur de fonctionnement de l'équipement de traitement connecté est allumé ;
des moyens de commande (55) pour commander l'activation des moyens de sortie en se basant sur
les résultats de détermination de la connexion et du type d'équipement de traitement obtenus par les moyens de détermination de l'équipement de traitement, et
la détection d'un interrupteur allumé par les moyens de détection de commutation ; et
des moyens de communication (54) pour envoyer les informations de commande d'activation du dispositif de scalpel électrique et les informations d'activation synchronisée avec le dispositif de scalpel électrique à d'autres appareils médicaux, et pour recevoir également les informations de commande d'activation et les informations d'activation synchronisée d'autres appareils médicaux, sous le contrôle des moyens de commande.

7. Système chirurgical selon la revendication 5, le dispositif de production d'ultrasons (2) comprenant :
des moyens de sortie (61) pour transmettre en sortie des signaux d'activation ultrasonores correspondant à l'équipement de traitement connecté à ceux-ci ;
des moyens de détermination de l'équipement de traitement (62) pour déterminer la connexion et le type d'équipement de traitement connecté ;
des moyens de détection de commutation (63) pour détecter qu'un interrupteur de fonctionnement de l'équipement de traitement connecté est allumé ;
des moyens de commande (65) pour commander l'activation des moyens de sortie en se basant sur
les résultats de détermination de la connexion et du type d'équipement de traitement obtenus par les moyens de détermination de l'équipement de traitement, et
la détection d'un interrupteur allumé par les moyens de détection de commutation ; et
des moyens de communication (64) pour envoyer les informations de commande d'activation du dispositif de scalpel électrique et les informations d'activation synchronisée avec le dispositif de production d'ultrasons à d'autres appareils médicaux, et pour recevoir également les informations de commande d'activation et les informations d'activation synchronisée d'autres appareils médicaux, sous le contrôle des moyens de commande.

8. Système chirurgical selon la revendication 5, le dispositif d'alimentation en eau/d'aspiration (3) comprenant :
une pompe d'alimentation en eau (71) et une pompe d'aspiration (72) ;
des moyens de détection de commutation (3) pour détecter qu'un interrupteur indiquant l'action d'activation de la pompe d'alimentation en eau et de la pompe d'aspiration est allumé ;
des moyens de commande (75) pour commander l'activation de la pompe d'alimentation en eau et de la pompe d'aspiration en se basant sur les instructions d'activation de la pompe d'alimentation en eau et de la pompe d'aspiration détectées par les moyens de détection de commutation ; et
des moyens de communication (74) pour envoyer les informations de commande d'activation du dispositif d'alimentation en eau/d'aspiration à d'autres appareils médicaux, et pour recevoir également les informations de commande d'activation et les informations d'activation synchronisée en provenance d'autres appareils médicaux, sous le contrôle des moyens de commande.

9. Système chirurgical selon la revendication 5, dans lequel le dispositif de pneumopéritoine (4) comprend :
une pompe d'évacuation d'air (81) et une pompe d'alimentation en air (82) ;
des moyens de détection de commutation (83) pour détecter qu'un interrupteur indiquant l'action d'activation de la pompe d'alimentation en air et de la pompe d'évacuation d'air est allumé ;
des moyens de commande (85) pour commander l'activation de la pompe d'alimentation en air et de la pompe d'évacuation en se basant sur les instructions d'activation de la pompe d'alimentation en air et de la pompe d'évacuation d'air détectées par les moyens de détection de commutation ; et
des moyens de communication (84) pour envoyer les informations de commande d'activation du dispositif de pneumopéritoine à d'autres appareils médicaux, et pour recevoir également les informations de commande d'activation et les informations d'activation synchronisée d'autres appareils médicaux, sous le contrôle des moyens de commande.
